# EUROPEAN PATENT SPECIFICATION

(11) **EP 1 852 131 B1**
(45) Date of publication and mention of the grant of the patent: **27.10.2010**
(21) Application number: 07251878.0
(22) Date of filing: 04.05.2007
(51) Int. Cl.: A61L 2/04, A61L 2/07, B67D 1/07

(54) **Water dispenser with disinfection circuit**
Wasserspender mit Desinfektionskreislauf
Distributeur d'eau avec un circuit de désinfection

(30) Priority: 06.05.2006 GB 0608968
(43) Date of publication of application: 07.11.2007
(73) Proprietor: IMI Cornelius (UK) Limited, Brighouse, West Yorkshire HO6 4LX (GB)
(72) Inventor: Wiemer, Klaus, 42799 Leichlingen (DE); Altenbach, Heinz, 51379 Leverkusen (DE)
(74) Representative: Wightman, David Alexander

(56) References cited:
- WO-A-95/11854
- WO-A-2005/068349
- CA-A1- 2 101 212
- DE-C- 80 938
- US-A- 5 290 442
- US-A- 5 766 453
- US-B1- 6 207 046

## Description

This invention relates to water dispensers and in particular to bottled waster dispensers as set from in the preamble of claim 1. Such a dispenser is known from US-A-6 201 046. More specifically, the invention concerns disinfection of bottled water dispensers to improve bacteriological quality of the dispensed water.

Bottled water dispensers for dispensing chilled water are well known in which a reservoir is supplied with water from a bottle that is inverted to connect the bottle to an inlet of the reservoir. In use, water in the reservoir is cooled to a desired temperature for dispense and delivered to an outlet for dispense into a glass, cup or similar vessel. The dispensed water is replaced by water from the bottle and, when the bottle is empty it can be detached and replaced by a full bottle.

A problem with these types of dispensers is that the water contained in the bottle contains bacteria which are introduced into the dispenser where the bacteria can rapidly grow with the result that the bacteriological quality of the dispensed water deteriorates with time. This can also lead to formation of a bio-film on internal surfaces of the dispenser that leads to rapid growth of bacteria when the bottle is removed and replaced by a new bottle.

US-5766453-A discloses a system that employs reverse osmosis purification or a cartridge filter arrangement

CA-2101212-A1 disposes a system in which a refrigerated water container directly supplies a cold water tank and a hot water tank for subsequent dispense. A pipe connects the tanks for circulating hot water in a sterilisation process independently of the flow delivery lines.

WO-2005/06834-A discloses a system in which a hot water tank and cold water tank are directly supplied by a water container and a pipe connects the tanks circulating hot water in a sterilisation process independently of the hot water flow delivery line.

US-5290442-A discloses a system in which a cold water tank is filled by connection to a mains supply. The tank can be kept in the fridge for dispense of cold water that is filtered before delivery.

WO-95/11854-A discloses a system in which a cleaning fluid is introduced and circulated in the system.

DE-80938-C discloses a system in which a cleaning/rinsing agent is introduced to the system.

The present invention has been made from a consideration of the foregoing problem.

In one aspect, the invention provides a bottled water dispenser as defined in claim 1. By this aspect of the invention the bacteriological quality of the dispensed water can be improved by disinfection of internal surfaces of the dispenser.

In one arrangement, the disinfection cycle can be activated automatically, for example with a programmable electronic controller or microprocessor. In one embodiment, disinfection is effected using steam or hot water generated in the hot water reservoir to disinfect the cold water reservoir. In another embodiment, disinfection is effected using steam or hot water generated in an auxiliary heater located in the circulation flow line to disinfect the cold water reservoir. In yet another embodiment, disinfection is effected using steam or hot water generated in the hot water reservoir and an auxiliary heater located in the circulation flow line to disinfect the cold water reservoir. In these embodiments, the circulation flow line is preferably connected to a cooling means whereby the hot water or steam can be cooled at the end of the disinfection cycle before re-activating a refrigeration circuit for cooling the water in the cold water reservoir.

Advantageously, the dispense flow line from the cold water reservoir includes a micro-filter to remove bacteria contained in the water prior to dispense. Preferably, the micro-filter is detachable and can be replaced at pre-determined intervals to maintain effectiveness of the filter. For example, replacement of the filter may be determined according to the length of time the filter has been in place or the volume of water that has passed through the filter. Appropriate sensors may be employed to provide a warning when the filter needs to be replaced. Such warning may include a visual and/or audible warning at the dispenser. Alternatively or additionally, such warning may be transmitted to a remote monitoring station to initiate a service call by a maintenance engineer to replace the filter. Preferably, the flow line includes a by-pass for the filter to reduce the amount of water that is re-circulated through the filter during the disinfection cycle. In this way, the life of the filter may be extended.

Preferably, the dispense outlets are adapted to inhibit growth of bacteria back into the dispense system caused by contamination of the outlets from human contact or from some other source such as airborne bacteria. For example, the dispense outlets may be provided with a coating of silver ions and/or exposed to UV radiation to kill bacteria coming into contact with the outlets. The outlets may be configured as described in our European patent application No.1440941.

The dispenser can dispense cold or hot water as desired. The dispensed water may be mixed with other beverage ingredients to produce a desired cold or hot beverage. The other ingredients may be added separately to the receptacle into which the water is dispensed by the user. Alternatively, the dispenser may be adapted to mix selected ingredients with the water for dispense of the desired beverage, for example by user selection at an interface panel. The dispense of water may be manually controlled or automatically controlled. For example a manually operable dispense valve may be employed or an electrically operable valve may be employed with a flowmeter or timer to dispense a known volume of water.

In another aspect, the invention provides a method for disinfecting the internal surfaces of a bottled water dispenser with steam or hot water as defined in claim 12. Such a method inhibits growth of bacteria in use of the dispenser.

In one arrangement, steam or hot water generated in the dispenser can be employed during the disinfection cycle.

The invention will now be described in more detail, by way of example only, with reference to the accompanying drawings wherein:
Figure 1 is a schematic view of a bottled water dispenser not according to the invention;
Figure 2 is a schematic view of a bottled water dispenser not according to the invention;
Figure 3 is a schematic view of a bottled water dispenser according to a first embodiment of the invention;
Figure 4 is a schematic view of a bottled water dispenser according to a second embodiment of the invention; and
Figure 5 is a schematic view of a bottled water dispenser according to a third embodiment of the invention

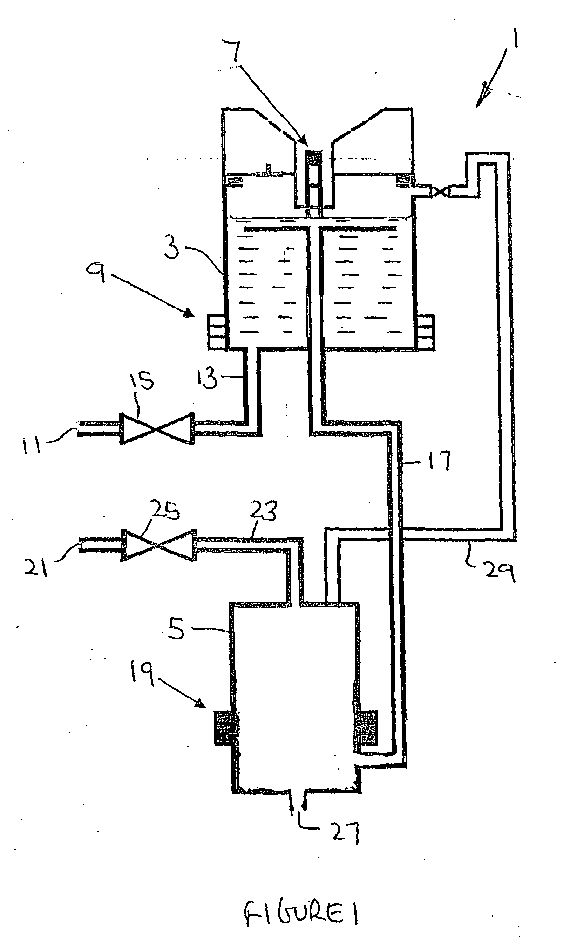

Referring first to Figure 1 of the accompanying drawings, a bottled water dispenser 1 not according to the invention is shown having a water dispensing system including a cold water reservoir 3 and a hot water reservoir 5.

The cold water reservoir 3 is configured at the upper end to support a bottle of water (not shown) in an inverted position with an outlet from the bottle detachably connected to an inlet 7 for supplying water to the cold water reservoir 3. The inlet 7 is arranged to admit water to the cold water reservoir 3 and air to the bottle when the water level in the cold water reservoir 3 falls below a pre-determined level. In this way, water dispensed from the system is automatically replenished with water from the bottle. The bottle is typically made of clear glass or plastics and is of a size that can be readily handled, for example, the bottle may contain five gallons (approximately 20 litres) of water. It will be understood that the size (volume) of the bottle may be changed.

Normally, the bottle will be visible so that the level of water in the bottle can be inspected and the bottle removed when empty and replaced by a full bottle. It will be understood this is not essential, however, and other means of monitoring the water level in the bottle can be employed to generate a signal when the bottle needs to be replaced. The signal may be visual and/or audible.

The water in the cold water reservoir 3 is cooled by any suitable means such as a refrigeration circuit including an evaporator 9 wrapped around the reservoir 3 to maintain the water in the reservoir 3 at a desired temperature for dispense such as 5-10 °C. For example, the temperature of the water in the reservoir 3 may be monitored by a temperature sensor (not shown) with operation of the refrigeration circuit being controlled in response to the temperature of the water in the reservoir 3.

The cold water reservoir 3 is connected to a dispense outlet 11 via a water flow line 13 including an on/off valve 15 for controlling dispense of cold water from the outlet 11 into a receptacle such as a glass or cup placed under the outlet 11. The valve 15 may be an electrically operated valve, for example a solenoid valve, controlled via a user interface panel (not shown) on the dispenser. Alternatively, the valve may be a manually operated valve controlled via a handle or lever accessible to the user.

The cold water reservoir 3 is also connected to the hot water reservoir 5 via a water flow line 17 for supplying cold water to the hot water reservoir 5. The water in the hot water reservoir 5 is heated by any suitable means such as a heating circuit including an electrical heater 19 wrapped around the reservoir 5 to maintain the water in the reservoir 5 at a desired temperature for dispense such as 70-90°C. For example, the temperature of the water in the reservoir 5 may be monitored by a temperature sensor (not shown) with operation of the heating circuit being controlled in response to the temperature of the water in the reservoir 5.

The hot water reservoir 5 is connected to a dispense outlet 21 via a water flow line 23 including an on/off valve 25 for controlling dispense of hot water from the outlet 21 into a receptacle such as a glass or cup placed under the outlet 21. The valve 25 may be an electrically operated valve, for example a solenoid valve, controlled via a user interface panel (not shown) on the dispenser. Alternatively, the valve may be a manually operated valve controlled via a handle or lever accessible to the user.

The hot water reservoir 5 is also provided with a drain outlet 27 having a valve (not shown) that is normally closed and can be opened when it is desired to empty the water dispenser 1. The drain valve may be a manually operable valve or an electrically operable valve. A water overflow line 29 connects the hot water reservoir 5 to the space above the water level in the cold water reservoir 3 to equalise the pressure in the system.

The water dispenser 1 can dispense cold or hot water as desired. The dispensed water may be mixed with other beverage ingredients to produce a desired cold or hot beverage. The other ingredients may be added separately to the receptacle into which the water is dispensed by the user.

Alternatively, the dispenser may be adapted to mix selected ingredients with the water for dispense of the desired beverage, for example by user selection at an interface panel.

In use, the bacteriological quality of the dispensed water is affected by the levels of bacteria in the stored water contained in the bottle and in the water dispensing system of the water dispenser 1. If left uncontrolled, rapid growth of bacteria can occur leading to off-taste and health risks when drinking the dispensed water, especially from the cold water reservoir where bio-film can build-up on internal surfaces of the reservoir 3 and the water flow lines to the outlet 11 and to the hot water reservoir 5. Growth of bacteria is less of a problem in water dispensed from the hot water reservoir 5 where the water is heated and maintained at an elevated temperature sufficient to inhibit growth or kill bacteria already present in the water.

Growth of bacteria in the water dispensing system can be controlled by carrying out a disinfection cycle using steam generated in the hot water reservoir 5. For this, the water bottle is detached from the inlet 7 and a closure cap fitted to the inlet 7. The cooling circuit for the cold water reservoir 3 is de-activated and water is drained from the cold water reservoir 3 and the flow lines 13,23. The hot water reservoir 5 is partially emptied to leave a space above the water level in the hot water reservoir 5. The dispense outlets 11 and 21 are then temporarily connected with a length of tubing (not shown) or the like and the valves 15,25 opened.

The disinfection cycle is then activated, for example via a control panel located within the dispenser 1 or otherwise adapted for restricted access and operation by authorised personnel only. During the disinfection cycle, water in the hot water reservoir is heated to generate steam that is then circulated in the system. The heating circuit for the hot water reservoir 5 may be provided with an auxiliary heater (not shown) to boost temporarily the power input for heating rapidly the water in the hot water reservoir 5 to generate the steam. The steam contacts the internal surfaces of the flow lines, the cold water reservoir and the inlet to render any bio-film on the surfaces inactive. The duration of the disinfection cycle may be controlled manually or automatically, for example by a timer control circuit to circulate steam for a pre-determined length of time. On completion of the disinfection cycle, the heating circuit is switched off, the valves 15,25 closed, the tubing connecting the outlets 11,21 removed, and the water bottle or a new water bottle connected to the inlet 7 to re-fill the water dispensing system. The disinfection cycle may be carried out at regular intervals, for example by a service technician.

In a modification (not shown), steam for the disinfection cycle may be generated by heating water in an auxiliary heater temporarily connected between the outlets 11,21 during the disinfection cycle.

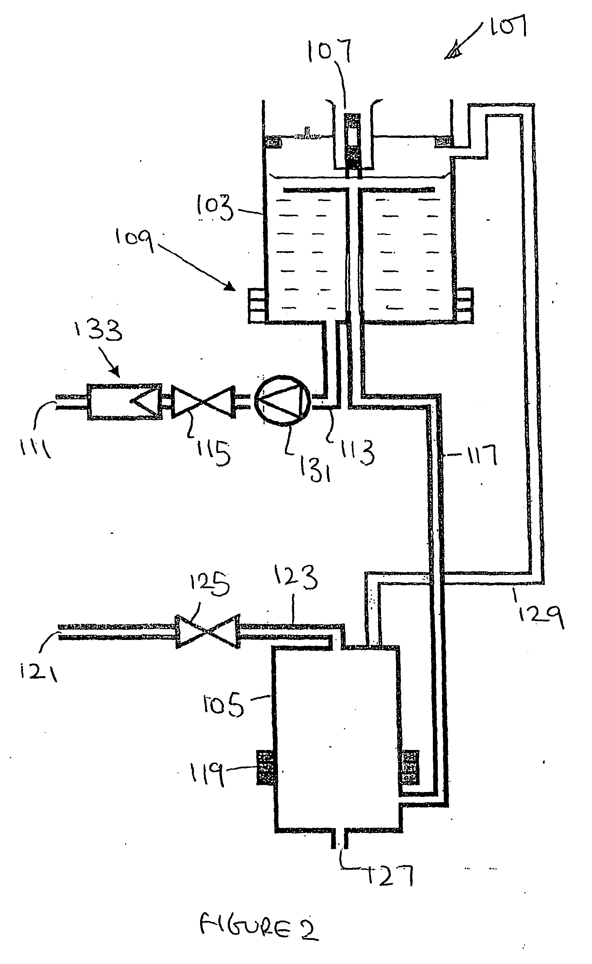

Referring now to Figure 2, another bottled water dispenser 101 not according to the invention is shown in which like reference numerals in the series 100 are used to indicate parts corresponding to the first embodiment.

In this dispenser 101, the water flow line 113 from the cold water reservoir 103 to the dispense outlet 111 includes a pump 131 and a micro-filter 133. The pump 131 is operable during the disinfection cycle described later and the micro-filter 133 is operable to remove bacteria from water dispensed via the outlet 111 from the cold water reservoir 103. By removing bacteria from the dispensed water, the bacteriological quality of the dispensed water is improved. The micro-filter 133 may be of any suitable type such as a membrane filter available from Filtrix. A micro-filter may be provided in the flow line from the cold water reservoir in the first embodiment described above.

The disinfection cycle is effected by circulating hot water around the water dispensing system. This may be carried out with the water bottle in situ or with the water bottle detached and the closure cap fitted to the inlet 107. For this, the dispense outlets 111,121 are again temporarily connected by a length of tubing (not shown) which includes an auxiliary heater (not shown) for heating water flowing through the tubing. In this embodiment, the auxiliary heater comprises a heat exchanger tank containing an electrical heating element for heating the water as it passes through the tank. Typically, the auxiliary heater will have a higher power rating than the heating circuit for the hot water reservoir to ensure rapid heating of the water to the required temperature for the disinfection cycle. In this way, the time for the water to reach the required temperature can be reduced. Thus, where the disinfection cycle is carried out by a service technician, the on-site time of the service technician may be reduced.

The disinfection cycle is then activated, for example via a control panel located within the dispenser 101 or otherwise adapted for restricted access and operation by authorised personnel only. During the disinfection cycle, the cooling circuit for the cold water reservoir 103 is switched off, the valves 115,125 are opened and the pump 131 is operable to circulate water around the water dispensing system from the cold water reservoir 103 to the hot water reservoir 105 and back to the cold water reservoir 103. The pump 131 may operate continuously during the disinfection cycle. Alternatively, the pump 131 is operable intermittently, i.e. cycles between on and off conditions, during the disinfection cycle. The times the pump is on and off may be preset or responsive to a sensor according to the operating conditions of the disinfection cycle. The circulating water is heated to an elevated temperature by the heater temporarily connected between the dispense outlets 111,121 via the tubing and is circulated around the water dispensing system for a time sufficient to kill bacteria in the water and render any bio-film on the internal surfaces inactive. The duration of the disinfection cycle may be controlled manually or automatically, for example by a timer control circuit to circulate steam for a pre-determined length of time. On completion of the disinfection cycle, the pump 131 and the auxiliary heater between the outlets 111,121 are switched off, the valves 115,125 closed and the tubing connecting the outlets 111,121 removed together with the auxiliary heater. The hot water remaining in the cold water reservoir 103 at the end of the disinfection cycle may be left to cool down naturally before re-activating the refrigeration circuit to cool the water to the desired dispense temperature. Alternatively, the outlet 111 may be opened to dispense the hot water from the cold water reservoir 103 and re-fill the cold water reservoir from the water bottle connected to the inlet 107. The disinfection cycle may be carried out at regular intervals, for example by a service technician.

In a modification (not shown), the external heater connected between the outlets for heating the water during the disinfection cycle may be omitted and the water heated to the required temperature in the hot water reservoir 105. The heating circuit for the hot water reservoir 105 may be provided with an auxiliary heater to boost temporarily the power input for heating rapidly the water in the hot water reservoir 105 for the disinfection cycle.

In the above-described embodiments, the disinfection cycle requires the presence of a service technician or the like to set up the water dispenser for the disinfection cycle by temporarily connecting the outlets from the hot and cold reservoirs.

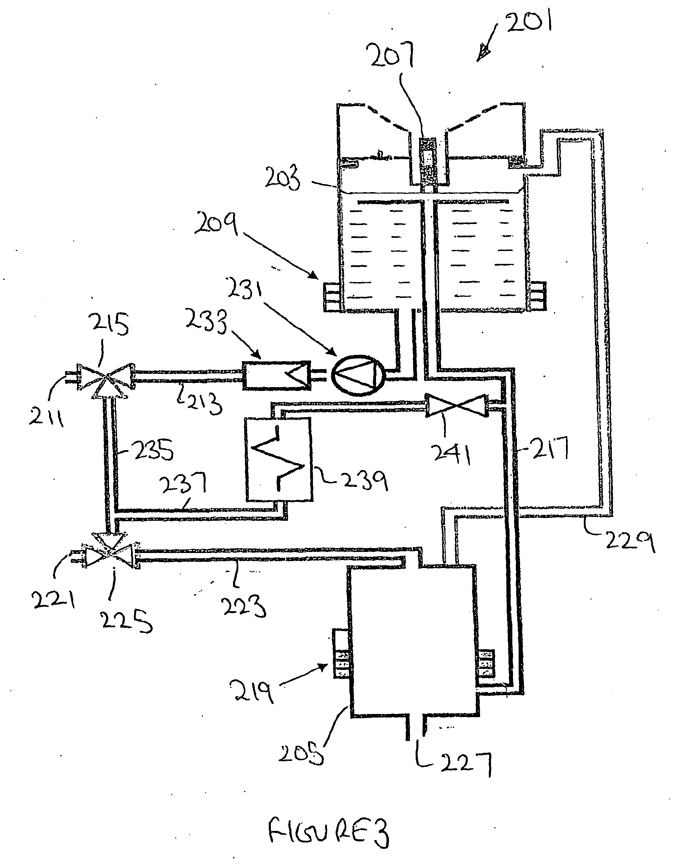

With reference now to Figure 3, a first embodiment of a bottled water dispenser 201 according to the invention is shown in which like reference numerals in the series 200 are used to indicate parts corresponding to the previous embodiments.

In this embodiment the water dispensing system is configured to enable the disinfection cycle to be automatically activated, for example overnight by employing three-way valves 215,225 in the flow lines 213,223 that can be switched between a dispense position for delivering water from the reservoirs 203,205 to the respective outlets 211,221 for dispense, and a disinfection position for circulating hot water around the water dispensing system via a water flow line 235 connecting the flow lines 213,223 during the disinfection cycle. In the dispense position, the valves 215,225 can be open or closed. The hot water for the disinfection cycle is generated in the hot water reservoir 205 and the heating circuit for the hot water reservoir 205 may be provided with an auxiliary heater to boost temporarily the power input for heating rapidly the water in the hot water reservoir 105 for the disinfection cycle.

At the end of the disinfection cycle, the water heating circuit is switched off and the circulating water is diverted through an air cooled heat exchanger 239 by switching valve 225 to the dispense position and opening an on-off valve 241 in a water flow line 237 extending between the flow line 235 and the flow line 217. In this way, the water circulating through the cold water reservoir 203 is cooled down before the refrigeration circuit is switched back-on. The on/off valve 241 is closed during the disinfection cycle and is open during the cool-down cycle.

A programmable controller (not shown) is provided for controlling the disinfection cycle such that the timing and duration of the disinfection cycle can be set to take place automatically, for example overnight.

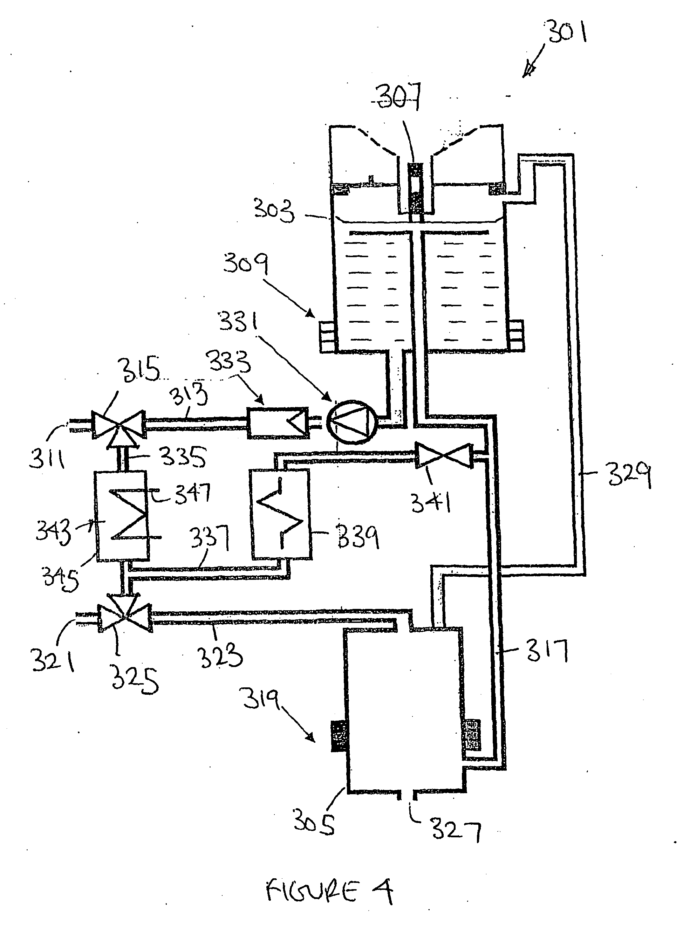

With reference now to Figure 4, a second embodiment of a bottled water dispenser 301 according to the invention is shown in which like reference numerals in the series 300 are used to indicate parts corresponding to the previous embodiments.

In this embodiment, an auxiliary heater 343 is provided in the flow line 335 for heating the circulating water during the disinfection cycle. The heater 343 comprises a heat exchanger tank 345 containing an electrical heating element 347 for heating the water as it flows through the tank 345. Typically, the auxiliary heater will have a higher power rating than the heating circuit for the hot water reservoir to ensure rapid heating of the water to the required temperature for the disinfection cycle. In other respects the construction and operation of this embodiment is similar to and will be understood from the description of Figure 3.

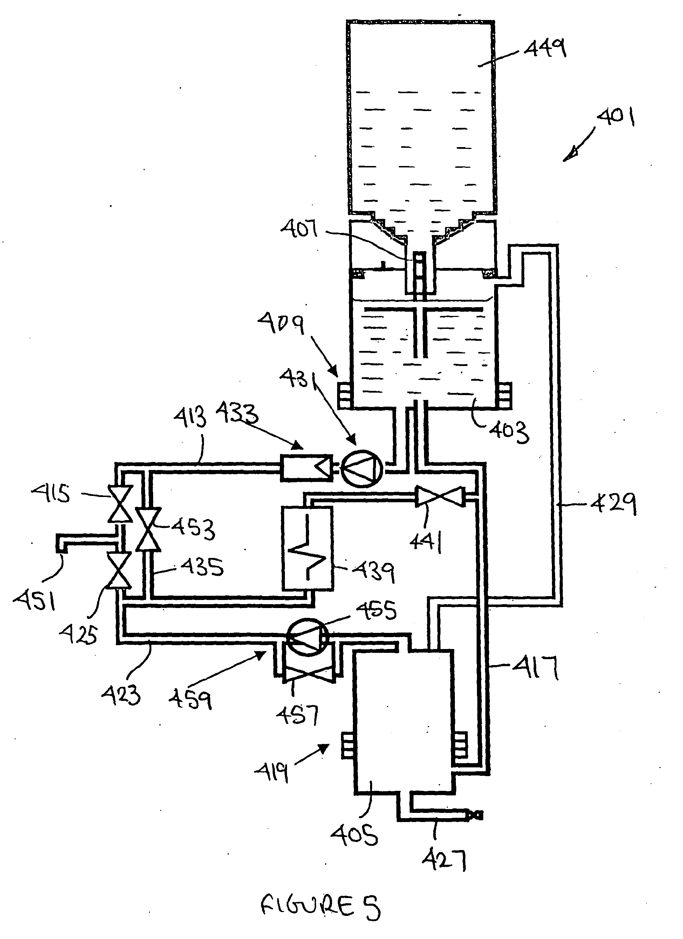

With reference now to Figure 5, a third embodiment of a bottled water dispenser 401 according to the invention is shown in which like reference numerals in the series 400 are used to indicate parts corresponding to the previous embodiments.

In this embodiment, a water bottle 449 is shown inverted and connected to the inlet 407 of the cold water reservoir. The flow lines 413,423 are connected to a common outlet 451 for selectively dispensing cold or hot water according to actuation of the valves 415,425 during normal operation of the water dispenser in the dispense mode in which on-off valve 441 in flow line 437 is closed and an on-off valve 453 in the flow line 435 connecting the flow lines 413,423 is closed. Pump 431 in cold water flow line 413 and a pump 455 in hot water flow line 423 may be operable in response to actuation of a dispense to assist water flow during dispense.

During the disinfection cycle in the cleaning or sanitisation mode, the valves 415,425 are closed and valve 453 is opened to connect the flow lines 413,423. Valve 441 is also closed and water heated in the hot water reservoir 405 to a temperature sufficient to kill bacteria is circulated around the system by operating pump 431 continuously or intermittently as described previously. Alternatively or additionally, an auxiliary heater (not shown) may be employed in the circulation flow line 435 to heat the water during the disinfection cycle. During this operation, pump 455 is switched-off and a valve 457 in by-pass line 459 opened to allow circulating water to by-pass the pump 455. A similar by-pass may be provided for the micro-filter 433 to reduce the amount of water flowing through the filter 433 during the disinfection cycle and thereby extend the life of the filter. On completion of the disinfection cycle, the valve 441 is opened to circulate the hot water through the heat exchanger 439 to cool the water before re-activating the cooling circuit to chill the water in the cold water reservoir 403. In other respects the operation of this embodiment is similar to and will be understood from the description of Figures 3 and 4.

In a modification (not shown), the first to third embodiments of the bottled water dispenser may be adapted so as to be operable to carry out the disinfection cycle using steam generated in the hot water reservoir and/or in the auxiliary heater where provided.

As will now be understood, the present invention provides a bottled water dispenser for dispensing hot or cold water in which growth of bacteria in the dispensed water can be controlled to improve the bacteriological quality of the dispensed water by regular disinfection of the water dispensing system with hot water or steam circulated in the system and/or by removal of bacteria from the water by a micro-filter.

In the above-described embodiments, the circulation of steam or hot water during the disinfection cycle is effective to render inactive any bio-film that has developed on the internal surfaces of the system. In this way, the rate of growth of bacteria in the water between successive disinfection cycles is reduced thereby improving the bacteriological quality of the dispensed water. In the embodiments of Figures 3 to 5, the dispenser has a built-in disinfection unit and can be programmed to carry out the disinfection cycle automatically when required. As a result, the water dispenser can be regularly disinfected, for example overnight, such that undesirable formation of a bio-film on the internal surfaces of the system is inhibited thereby reducing any build-up of bio-film leading to an improvement in water quality. Nevertheless, all the above-described embodiments may benefit from an active sanitisation with chemicals at pre-determined intervals to remove completely any bio-film that has collected on the internal surfaces of the system. The time interval between such active sanitisations may be increased in the embodiments with built-in disinfection units which can be operated without the presence of service personnel.

Various improvements and modifications of the above-described bottled water dispensers will be apparent to those skilled in the art.

For example, the outlets may be adapted to prevent growth of bacteria back into the water dispense system. Thus, the outlets may be coated with silver ions and/or be exposed to a UV source to kill bacteria present from human contact with the outlets or from some other source of bacteria. Alternatively or additionally, we may provide a UV source in the cold water reservoir or elsewhere (e.g. in the bottle) to kill bacteria present in the water.

It will also be understood that features of the various embodiments described herein may be employed separately or in combination with features of any other embodiments. For example, pumps provided in the cold and hot water flow lines of Figure 5 to assist dispense may be employed in any of the other embodiments. Alternatively, the pump in the hot water flow line of Figure 5 may be omitted and the pump in the cold water flow line used during the disinfection cycle only as described in the other embodiments. A single outlet for hot and cold water as shown in Figure 5 may be employed in any of the other embodiments.

## Claims

1. A bottled water dispenser including a water dispense system for releasable connection to a bottle (449) containing water to be dispensed, the system comprising a cold water reservoir (203; 303; 403) adapted to provide cold water, a hot water reservoir (205; 305; 405) comprising means for heating the water (219; 319; 419) adapted to provide hot water, a dispense flow line (213;313;413) from the cold water reservoir (203;303;403) to an outlet (211;311;411) for dispensing cold water, a dispense flow line (223;323;423) from the hot water reservoir to an outlet (221;321;421) for dispensing hot water, **characterised in that** the cold water reservoir (203;303;403) is configured at an upper end to support a bottle (449) of water in an inverted position with an outlet from the bottle (449) detachably connected to an inlet (207;307;407) of the cold water reservoir (203;303;403) for supplying water to the cold water reservoir (203;303;403); a supply line (217;317;417) connecting the cold water reservoir (203;303;403) to the hot water reservoir (205;305;405) for supplying water from the cold water reservoir (203;303;403) to the hot water reservoir (5;105;205;305;405), a circulation flow line (235;335;435) connecting the cold water dispense flow line (213;313;413) to the hot water dispense flow line (223;323;423), and valve means (215,225;315,325;453) for temporarily opening the circulation flow line (235;335;435) to connect the dispense flow lines (213,223;313,323;413,423) for circulating steam or hot water in the system during the disinfection cycle and for closing the circulation flow line (235;335;435).

2. A bottled water dispenser according to claim 1 **characterised in that** means is provided for controlling the disinfection cycle.

3. A bottled water dispenser according to claim 1 or claim 2 **characterised in that** an auxiliary heater (343) is located in the circulation flow line (335) for generating steam or hot water during the disinfection cycle.

4. A bottled water dispenser according to any preceding claim **characterised in that** the circulation flow line (235;335) is connected to cooling means (239;339) for cooling hot water or steam used for the disinfection cycle.

5. A bottled water dispenser according to any preceding claim **characterised in that** the dispense flow line (113;213;313) from the cold water reservoir (103;203;303) includes a micro-filter (133;233;333) to remove bacteria contained in the water prior to dispense.

6. A bottled water dispenser according to claim 5 **characterised in that** the micro-filter (133;233;333) is detachable and sensor means is employed to provide a warning when the filter needs to be replaced.

7. A bottled water dispenser according to claim 6 **characterised in that** a by-pass is provided for by-passing the filter during the disinfection cycle.

8. A bottled water dispenser according to any preceding claim **characterised in that** a pump (231;331;431) is provided in the cold water dispense flow line (213;313;413) for circulating water during the disinfection cycle.

9. A bottled water dispenser according to claim 8 wherein dispense of cold water during the normal mode of operation is assisted by the pump (231;331;431).

10. A bottled water dispenser according to any preceding claim **characterised in that** a pump (455) is provided in the hot water flow line for assisting dispense of hot water during the normal mode of operation.

11. A bottled water dispenser according to claim 10 wherein a by-pass is provided for by-passing the pump (455) during the disinfection cycle.

12. A method for disinfecting internal surfaces of a bottled water dispenser comprising the steps of providing a dispense system including a cold water reservoir (203;303) adapted for dispensing cold water via a flow line (213;313) having an outlet (211;311), and a hot water reservoir (205;305) comprising means for heating the water (219; 319; 419) adapted for dispensing hot water via a flow line (223;323) having an outlet (221, 321), **characterised by** steps of inverting a bottle (449) of water and releasably connecting an outlet of the bottle (449) to an inlet (207;307;407) of the cold water reservoir (203;303;403) for supplying water to the cold water reservoir (203;303;403), providing a supply line (217;317;417) connecting the cold water reservoir (203;303;403) to the hot water reservoir (205;305;405) for supplying water from the cold water reservoir (203;303;403) to the hot water reservoir (205;305;405), providing a circulation flow line (235;335;435) connecting the cold water flow line (213;313;413) to the hot water flow line (223;323;423), providing valve means (215,225;315,325;453) for temporarily opening the circulation flow line (235;335;435) to connect the flow lines (213,223;313,323;413,423) for circulating steam or hot water in the system during the disinfection cycle and for closing the circulation flow line (235;335;435).

13. A method according to claim 12 **characterised in that** an auxiliary heater (343) is employed to generate the steam or hot water used in the disinfection cycle.

14. A method according to 13 **characterised in that** the auxiliary heater (343) is connected between the flow lines ( 313,323).

15. A method according to any of claims 12 to 14 **characterised by** circulating steam or hot water used for the disinfection cycle through a cooling means (239;339;439) for cooling the steam or hot water at the end of the disinfection cycle.

## Patentansprüche

1. Wasserspender für in Flaschen befindliches Wasser mit einem Wasserausgabesystem für eine lösbare Verbindung an eine das auszugebende Wasser enthaltende Flasche (449), wobei das System ein Kaltwasserreservoir (203;303;403) umfasst, welches geeignet ist, Kaltwasser zu liefern, einem Heißwasserreservoir (205;305;405), welches Mittel (219;319;419) zum Erhitzen des Wassers umfasst, und geeignet ist, Heißwasser zu liefern, einer Ausgabeströmungsleitung (213;313;413) von dem Kaltwasserreservoir (203;303;403) zu einem Auslass (211;311;411) zum Ausgeben von kaltem Wasser, einer Ausgabeströmungsleitung (223;323;423) von dem Heißwasserreservoir zu einem Auslass (221;321;421) zur Ausgabe von Heißwasser, **dadurch gekennzeichnet, dass** das Kaltwasserreservoir (203;303;403) an einem oberen Ende zum Tragen einer Wasserflasche (449) in einer umgekehrten Position ausgebildet ist, wobei ein Auslass von der Flasche (449) abnehmbar an einem Einlass (207;307;407) des Kaltwasserreservoirs (203;303;403) angeschlossen ist, um Wasser zu dem Kaltwasserreservoir (203;303;403) zuzuführen, eine Versorgungsleitung (217;317;417), welche das Kaltwasserreservoir (203;303;403) mit dem Heißwasserreservoir (205;305;405 verbindet, um Wasser von dem Kaltwasserreservoir (203;303;403) zu dem Heißwasserreservoir (5;105;205;305;405) zuzuführen, eine Zirkulationsströmungsleitung (235;335;435), welche die Kaltwasserausgabeströmungsleitung (213;313;413) mit der Heißwasserausgabeströmungsleitung (223;323;423) verbindet, und Ventilmittel (215,225;315,325;453), um zeitweilig die Zirkulationsströmungsleitung (235;335;435) zu öffnen, um die Ausgabeströmungsleitungen (213,223;313,323;413,423) zu verbinden, um Dampf oder Heißwasser in dem System während des Desinfektionszyklus zirkulieren zu lassen und zum Schließen der Zirkulationsströmungsleitung (235;335;435).

2. Wasserspender für in Flaschen befindliches Wasser nach Anspruch 1, **dadurch gekennzeichnet, dass** ein Mittel vorgesehen ist, um den Desinfektionszyklus zu steuern.

3. Wasserspender für in Flaschen befindliches Wasser nach Anspruch 1 oder 2, **dadurch gekennzeichnet, dass** eine Hilfsheizung (343) in der Zirkulationsströmungsleitung (335) angeordnet ist, um während des Desinfektionszyklus Dampf oder Heißwasser zu erzeugen.

4. Wasserspender für in Flaschen befindliches Wasser nach einem der vorstehenden Ansprüche, **dadurch gekennzeichnet, dass** die Zirkulationsströmungsleitung (235;335) an ein Kühlmittel (239;339) angeschlossen ist, um Heißwasser oder Dampf, die für den Desinfektionszyklus verwendet wurden, zu kühlen.

5. Wasserspender für in Flaschen befindliches Wasser nach einem der vorstehenden Ansprüche, **dadurch gekennzeichnet, dass** die Ausgabeströmungsleitung (113;213;313) von dem Kaltwasserreservoir (103;203;303) einen Mikrofilter (133;233;333) umfasst, um im Wasser enthaltene Bakterien vor der Ausgabe zu entfernen.

6. Wasserspender für in Flaschen befindliches Wasser nach Anspruch 5, **dadurch gekennzeichnet, dass** der Mikrofilter (133;233;333) abnehmbar ist, und dass ein Sensormittel verwendet wird, um eine Warnung zu erzeugen, wenn der Filter ersetzt werden muss.

7. Wasserspender für in Flaschen befindliches Wasser nach Anspruch 6, **dadurch gekennzeichnet, dass** ein Bypass vorgesehen ist, um während des Desinfektionszyklus den Filter zu umgehen.

8. Wasserspender für in Flaschen befindliches Wasser nach einem der vorstehenden Ansprüche, **dadurch gekennzeichnet, dass** eine Pumpe (231;331;431) in der Kaltwasserausgabeströmungsleitung (213;313;413) vorgesehen ist, um das Wasser während des Desinfektionszyklus zu zirkulieren.

9. Wasserspender für in Flaschen befindliches Wasser nach Anspruch 8, wobei die Ausgabe von Kaltwasser während des Normalbetriebs durch die Pumpe (231;331;431) unterstützt ist.

10. Wasserspender für in Flaschen befindliches Wasser nach einem der vorstehenden Ansprüche, **dadurch gekennzeichnet, dass** eine Pumpe (455) in der Heißwasserströmungsleitung vorgesehen ist, um die Ausgabe von Heißwasser während des Normalbetriebs zu unterstützen.

11. Wasserspender für in Flaschen befindliches Wasser nach Anspruch 10, wobei ein Bypass vorgesehen ist, um während des Desinfektionszyklus die Pumpe (455) zu umgehen.

12. Verfahren zum Desinfizieren der Innenfläche eines Wasserspenders für in Flaschen befindliches Wasser, umfassend die Schritte der Bereitstellung eines Ausgabesystems, einschließend ein Kaltwasserreservoir (203;303), welches geeignet ist, um über eine Strömungsleitung (213;313), welche einen Auslass (211;311) aufweist, Kaltwasser auszugeben, und ein Heißwasserreservoir (205;305), welches Mittel (219;319;419) zum Erhitzen des Wassers umfasst und geeignet ist, Heißwasser über eine Strömungsleitung (223;323), die einen Auslass (221;321) aufweist, auszugeben, **gekennzeichnet durch** die Schritte, eine Flasche (449) mit Wasser umzustülpen und lösbar einen Auslass der Flasche (449) an einen Einlass (207;307;407) des Kaltwasserreservoirs (203;303) anzuschließen, um Wasser zu dem Kaltwasserreservoir (203;303;403) zu leiten, Bereitstellen einer Versorgungsleitung (217;317;417), welche das Kaltwasserreservoir (203;303;403) mit dem Heißwasserreservoir (205;305;405) verbindet, um Wasser von dem Kaltwasserreservoir (203;303;403) zu dem Heißwasserreservoir (205;305) zu leiten, Bereitstellen einer Zirkulationsströmungsleitung (235;335;435), welche die Kaltwasserströmungsleitung (213;313;413) mit der Heißwasserströmungsleitung (223;323;423) verbindet, Bereitstellen von Ventilmitteln (215,225;315,325;453), um zeitweilig die Zirkulationsströmungsleitung (235;335;435) zu öffnen, um die Strömungsleitungen (213,223;313,323;413;423) zum Zirkulieren von Dampf oder Heißwasser in dem System während des Desinfektionszyklus zu öffnen, und um die Zirkulationsströmungsleitung (235;335;435) zu schließen.

13. Verfahren nach Anspruch 12, **dadurch gekennzeichnet, dass** eine Hilfsheizung (343) verwendet wird, um den Dampf oder das Heißwasser zu erzeugen, welches in dem Desinfektionszyklus verwendet wird.

14. Verfahren nach Anspruch 13, **dadurch gekennzeichnet, dass** die Hilfsheizung (343) zwischen den Strömungsleitungen (313;323) angeschlossen ist.

15. Verfahren nach einem der Ansprüche 12 bis 14, **dadurch gekennzeichnet, dass** der Dampf oder das Heißwasser, welche für den Desinfektionszyklus verwendet werden, durch ein Kühlmittel (239;339;439) zirkuliert werden, um den Dampf oder das Heißwasser am Ende des Desinfektionszyklus abzukühlen.

## Revendications

1. Distributeur d'eau en bouteille comprenant un système de distribution d'eau pour le raccordement amovible à une bouteille (449) contenant l'eau à distribuer, le système comprenant un réservoir d'eau froide (203; 303; 403) adapté pour fournir de l'eau froide, un réservoir d'eau chaude (205; 305; 405) comprenant des moyens pour chauffer l'eau (219; 319; 419), adapté pour fournir de l'eau chaude, une conduite d'écoulement de distribution (213; 313; 413) du réservoir d'eau froide (203; 303; 403) à une sortie (211; 311; 411) pour distribuer de l'eau froide, une conduite d'écoulement de distribution (223; 323; 423) du réservoir d'eau chaude à une sortie (221; 321; 421) pour distribuer de l'eau chaude, **caractérisé en ce que** le réservoir d'eau froide (203; 303; 403) est configuré au niveau d'une extrémité supérieure pour supporter une bouteille (449) d'eau dans une position inversée avec une sortie à partir de la bouteille (449) raccordée de manière détachable à une entrée (207; 307; 407) du réservoir d'eau froide (203; 303; 403) pour alimenter l'eau au réservoir d'eau froide (203; 303; 403); une conduite d'alimentation (217; 317; 417) raccordant le réservoir d'eau froide (203; 303; 403) au réservoir d'eau chaude (205; 305; 405) pour alimenter l'eau du réservoir d'eau froide (203; 303; 403) au réservoir d'eau chaude (5; 105; 205; 305; 405), une conduite d'écoulement de circulation (235; 335; 435) raccordant la conduite d'écoulement de distribution d'eau froide (213; 313; 413) à la conduite d'écoulement de distribution d'eau chaude (223; 323; 423), et des moyens de soupape (215, 225; 315, 325; 453) pour ouvrir temporairement la conduite d'écoulement de circulation (235; 335; 435) afin de raccorder les conduites d'écoulement de distribution (213, 223; 313, 323; 413, 423) pour faire circuler de la vapeur ou de l'eau chaude dans le système pendant le cycle de désinfection et pour fermer la conduite d'écoulement de circulation (235; 335; 435).

2. Distributeur d'eau en bouteille selon la revendication 1, **caractérisé en ce que** l'on prévoit des moyens pour contrôler le cycle de désinfection.

3. Distributeur d'eau en bouteille selon la revendication 1 ou la revendication 2, **caractérisé en ce qu'**un dispositif de chauffage auxiliaire (343) est positionné dans la conduite d'écoulement de circulation (335) pour générer de la vapeur ou de l'eau chaude pendant le cycle de désinfection.

4. Distributeur d'eau en bouteille selon l'une quelconque des revendications précédentes, **caractérisé en ce que** la conduite d'écoulement de circulation (235; 335) est raccordée à des moyens de refroidissement (239; 339) pour refroidir l'eau chaude ou la vapeur utilisée pour le cycle de désinfection.

5. Distributeur d'eau en bouteille selon l'une quelconque des revendications précédentes, **caractérisé en ce que** la conduite d'écoulement de distribution (113; 213; 313) à partir du réservoir d'eau froide (103; 203; 303) comprend un microfiltre (133; 233; 333) afin de supprimer les bactéries contenues dans l'eau avant la distribution.

6. Distributeur d'eau en bouteille selon la revendication 5, **caractérisé en ce que** le microfiltre (133; 233; 333) est détachable et des moyens de capteur sont utilisés pour fournir un avertissement lorsque le filtre doit être remplacé.

7. Distributeur d'eau en bouteille selon la revendication 6, **caractérisé en ce qu'**une dérivation est prévue pour contourner le filtre pendant le cycle de désinfection.

8. Distributeur d'eau en bouteille selon l'une quelconque des revendications précédentes, **caractérisé en ce qu'**une pompe (231; 331; 431) est prévue dans la conduite d'écoulement de distribution d'eau froide (213; 313; 413) pour faire circuler l'eau pendant le cycle de désinfection.

9. Distributeur d'eau en bouteille selon la revendication 8, dans lequel la distribution de l'eau froide pendant le mode de fonctionnement normal est assistée par la pompe (231; 331; 431).

10. Distributeur d'eau en bouteille selon l'une quelconque des revendications précédentes, **caractérisé en ce qu'**une pompe (455) est prévue dans la conduite d'écoulement d'eau chaude pour assister la distribution d'eau chaude pendant le mode de fonctionnement normal.

11. Distributeur d'eau en bouteille selon la revendication 10, dans lequel une dérivation est prévue pour contourner la pompe (455) pendant le cycle de désinfection.

12. Procédé pour désinfecter des surfaces internes d'un distributeur d'eau en bouteille comprenant les étapes consistant à prévoir un système de distribution comprenant un réservoir d'eau froide (203; 303) adapté pour distribuer de l'eau froide via une conduite d'écoulement (213; 313) ayant une sortie (211; 311), et un réservoir d'eau chaude (205; 305) comprenant des moyens pour chauffer l'eau (219; 319; 419), adapté pour distribuer l'eau chaude via une conduite d'écoulement (223; 323) ayant une sortie (221; 321), **caractérisé par** les étapes consistant à retourner une bouteille (449) d'eau et raccorder de manière amovible une sortie de la bouteille (449) à une entrée (207; 307; 407) du réservoir d'eau froide (203; 303; 403) pour alimenter l'eau au réservoir d'eau froide (203; 303; 403), prévoir une conduite d'alimentation (217; 317; 417) raccordant le réservoir d'eau froide (203; 303; 403) au réservoir d'eau chaude (205; 305; 405) pour alimenter l'eau du réservoir d'eau froide (203; 303; 403) au réservoir d'eau chaude (205; 305; 405), prévoir une conduite d'écoulement de circulation (235; 335; 435) raccordant la conduite d'écoulement d'eau froide (213; 313; 413) à la conduite d'écoulement d'eau chaude (223; 323; 423), prévoir des moyens de soupape (215, 225; 315, 325; 453) pour ouvrir temporairement la conduite d'écoulement de circulation (235; 335; 435) afin de raccorder les conduites d'écoulement (213, 223; 313, 323; 413, 423) pour faire circuler de la vapeur ou de l'eau chaude dans le système pendant le cycle de désinfection et pour fermer la conduite d'écoulement de circulation (235; 335; 435).

13. Procédé selon la revendication 12, **caractérisé en ce qu'**un dispositif de chauffage auxiliaire (343) est utilisé pour générer de la vapeur ou de l'eau chaude utilisée dans le cycle de désinfection.

14. Procédé selon la revendication 13, **caractérisé en ce que** le dispositif de chauffage auxiliaire (343) est raccordé entre les conduites d'écoulement (313, 323).

15. Procédé selon l'une quelconque des revendications 12 à 14, **caractérisé par** l'étape consistant à faire circuler de la vapeur ou de l'eau chaude utilisée pour le cycle de désinfection à travers des moyens de refroidissement (239; 339; 439) afin de refroidir la vapeur ou l'eau chaude à la fin du cycle de désinfection.
